# EUROPEAN PATENT SPECIFICATION

(11) **EP 1 949 893 B1**
(45) Date of publication and mention of the grant of the patent: **13.10.2010**
(21) Application number: 08153077.6
(22) Date of filing: 04.08.2005
(51) Int. Cl.: A61K 31/00, A61K 31/517, A61K 31/519, A61P 9/10

(54) **Quinazoline derivatives useful for the treatment of peripheral arterial disease and as phosphodiesterase inhibitors**
Chinazolinderivate als Phosphodiesterasehemmer zur Verwendung für die Behandlung peripherer Arterienerkrankungen
Dérives de quinazoline comme inhibiteurs de la phosphodiesterase utiles pour le traitement de la maladie artérielle périphérique

(30) Priority: 04.08.2004 US 598432 P
(43) Date of publication of application: 30.07.2008
(62) Divisional of application: 05784874.9
(73) Proprietor: Shire Biopharmaceuticals Holdings Ireland Limited, St Helier JE4 8PX, Jersey (GB)
(72) Inventor: Franklin, Richard, Fleet, Hampshire GU51 4NQ (GB)
(74) Representative: Harrison Goddard Foote

(56) References cited:
- WO-A-01/21163
- WO-A-2004/064841
- LANE W J ET AL: "Anagrelide metabolite induces thrombocytopenia in mice by inhibiting megakaryocyte maturation without inducing platelet aggregation" EXPERIMENTAL HEMATOLOGY 2001 US, vol. 29, no. 12, 2001, pages 1417-1424, XP002484397 ISSN: 0301-472X
- ERUSALIMSKY J D ET AL: "Is the platelet lowering activity of anagrelide mediated by its major metabolite 2-amino-5,6-dichloro-3,4-dihydroquinazolin e (RL603)?" EXPERIMENTAL HEMATOLOGY 2002 US, vol. 30, no. 7, 2002, pages 625-627, XP002484398 ISSN: 0301-472X
- RAFII SHAHIN ET AL: "Is the platelet lowering activity of anagrelide mediated by its major metabolite 2-amino-5,6-dichloro-3,4-dihydroquinazolin e (RL603)? In response" EXPERIMENTAL HEMATOLOGY (CHARLOTTESVILLE), vol. 30, no. 7, July 2002 (2002-07), pages 626-627, XP002484399 ISSN: 0301-472X
- OSINSKI M T ET AL: "Inhibition of platelet-derived growth factor-induced mitogenesis by phosphodiesterase 3 inhibitors: Role of protein kinase A in vascular smooth muscle cell mitogenesis" BIOCHEMICAL PHARMACOLOGY 20000801 US, vol. 60, no. 3, 1 August 2000 (2000-08-01), pages 381-387, XP002484400 ISSN: 0006-2952
- STALDER H: "METABOLITEN DER 1,5-DIHYDROIMIDAZO not 2,1-B 3/4 CHINAZOLIN-2(3H)-ONE. SYNTHESE UND REAKTIONEN EINIGER 1,5-DIHYDRO-3-HYDROXYIMIDAZO not 2,1-B 3/4 CHINAZOLIN-2(3H)-ONE METABOLITES OF 1,5-DIHYDROIMIDAZO not 2,1-B 3/4 QUINAZOLIN-2(3H)-ONES. PREPARATION AND REACTIONS OF SOME 1,5-DIHYDRO-3-HYDROXYIMIDAZO" HELVETICA CHIMICA ACTA, VERLAG HELVETICA CHIMICA ACTA. BASEL, CH, vol. 69, no. 8, 1 April 1986 (1986-04-01), pages 1887-1897, XP000984450 ISSN: 0018-019X
- KIENZLE F ET AL: "1,5-DIHYDROIMIDAZOQUINAZOLINONES AS BLOOD PLATELET AGGREGATION INHIBITORS" CHIMIE THERAPEUTIQUE, EDITIONS DIMEO, ARCUEIL, FR, vol. 17, no. 6, 1 January 1982 (1982-01-01), pages 547-556, XP009059097 ISSN: 0009-4374
- WANG GUOSU ET AL: "Comparison of the biological activities of anagrelide and its major metabolites in haematopoietic cell cultures" BRITISH JOURNAL OF PHARMACOLOGY, vol. 146, no. 3, October 2005 (2005-10), pages 324-332, XP002484401 ISSN: 0007-1188

## Description

### FIELD OF THE INVENTION

The present invention relates to compounds which are useful for inhibiting phosphodiesterase. More specifically, the present invention also provides compounds that are useful for treating or preventing Peripheral Arterial Disease.

### BACKGROUND OF THE INVENTION

Peripheral Arterial Disease is characterized by hardening of the arteries, or atherosclerosis, resulting from fatty deposits, especially in the blood vessels of the legs. This condition affects more than 10% of the adult population, primarily people over 55. Intermittent claudication is a symptom of peripheral arterial disease characterized by pain in the legs or buttocks during exercise. Intermittent claudication still represents a largely unmet medical need for which the only other major treatment, apart from surgical intervention with balloon angioplasty, appears to be oxpentifylline and hexopal -- both older and non-specific agents. Atherosclerosis is another name for "hardening of the arteries" and causes peripheral arterial disease. It is a disease process during which cholesterol deposits form in the walls of arteries. These fatty deposits restrict blood flow and decrease the delivery of oxygen and nutrients to the various organs and tissues of the body. When atherosclerosis occurs in leg arteries, it can lead to intermittent claudication.

Phosphodiesterases (PDEs) are a class of intracellular enzymes involved in the metabolism of the second messenger nucleotides cyclic adenosine monophosphate (cAMP) and cyclic guanosine monophosphate (cGMP) (see, e.g., Doherty, 1997, "Oral, Transdermal and Transurethral Therapies for Erectile Dysfunction" in Male Infertility and Dysfunction, Hellstrom, ed., Chapter 34, Springer-Verlag: New York). Numerous phosphodiesterase inhibitors have previously been described in the literature for a variety of therapeutic uses, including treatment of obstructive lung disease, allergies, hypertension, angina, congestive heart failure and depression (see, e.g., Goodman and Gilman's The Pharmacological Basis of Therapeutics, 1996, Ninth Edition, Chapter 34, McGraw-Hill: New York). PDEIII inhibition is associated with peripheral vasodilatation and anti-aggregatory, and hence, anti-thrombotic, effects.

A compound encompassed by a use of the present invention is 3-hydroxyanagrelide. This is a metabolite of anagrelide (see, for example, U.S. Patent Application Serial No. 10/762,566 published as U.S. Pub. No. 20040209907).

Various metabolites of anagrelide have been studied in the literature: Erusalimsky et al. (2002) Is the platelet lowering activity of anagrelide mediated by its major metabolite 2-amino-5,6-dichloro-3,4-dihydroquinazoline (RL603)? Exp Hematol. 30:625-7; Lane et al. (2001) Anagrelide metabolite induces thrombocytopenia in mice by inhibiting megakaryocyte maturation without inducing platelet aggregation. Exp Hematol. 29:1417-241; and Gaver et al. (1981) Disposition of anagrelide, an inhibitor of platelet aggregation. Clin Pharmacol Ther. 29:381-6.

It is among the objects of the present invention to provide alternative treatment to Peripheral Arterial Disease including intermittent claudication and to provide new phosphodiesterase inhibitors.

### SUMMARY OF THE INVENTION

One embodiment of this invention provides a compound of formula (II) 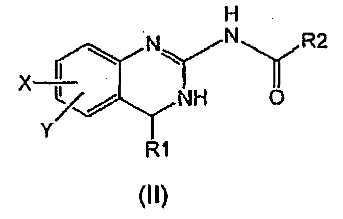 an equilibrating form thereof, a pharmaceutically acceptable salt thereof, or a pharmaceutically acceptable salt of an equilibrating form thereof, to a subject in need of such treatment, wherein X, Y, R1 and R2 are as defined below, for use in the treatment of peripheral arterial disease, atherosclerosis, intermittent claudication, conjestive heart failure, a disease state related to vasodilation or sexual dysfunction.

In one embodiment, the present invention provides a compound of formula 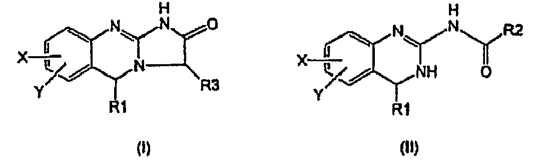 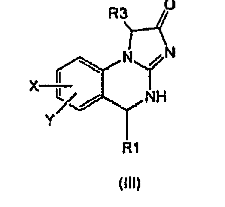 an equilibrating form thereof, a pharmaceutically acceptable salt thereof, or a pharmaceutically acceptable salt of an equilibrating form thereof,
wherein
R1 is H, C₁₋₆ alkyl, C₂₋₆ alkenyl, C₂₋₆ alkynyl, C₁₋₆ alkoxy, or C₆₋₁₀ aryl;
R2 is 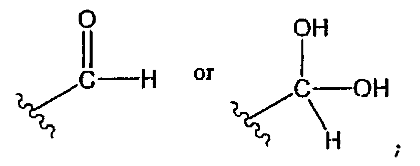 R3 is OH, halogen, SH, O-C₁₋₆ alkyl or NH₂; and
X and Y are independently H or halogen, for the uses of the invention.

Preferred of the above compounds include the X and Y substituents on the 5 and 6 positions of the benzyl ring.

The compounds of the invention may be used in methods for inhibiting a phosphodiesterase enzyme, treatment or prevention of Peripheral Arterial Disease, inhibiting platelet aggregation, and treatment or prevention of congestive heart failure comprising administering to a subject in need thereof a therapeutically effective amount of a compound of formulas (I) through (III).

There is also provided a pharmaceutical formulation comprising the compound of the invention in combination with a pharmaceutically acceptable carrier or excipient.

There is also provided a pharmaceutical formulation comprising at least one compound according to formulas (I) through (III) with at least one further therapeutic agent.

There is also provided the use of a compound of formulas (I) through (III) for the manufacture of a medicament for the treatment of Peripheral Arterial Disease.

### DETAILED DESCRIPTION OF THE INVENTION

Unless otherwise defined, all technical and scientific terms used herein have the same meaning as commonly understood by one of ordinary skill in the art to which this invention belongs. In case of conflict, the present specification, including definitions, will control. In addition, the materials, methods, and examples are illustrative only and not intended to be limiting.

In one embodiment of the present invention, compounds of the present invention comprise those wherein the following embodiments are present, either independently or in combination.

In one embodiment, the present invention provides a compound of the formula 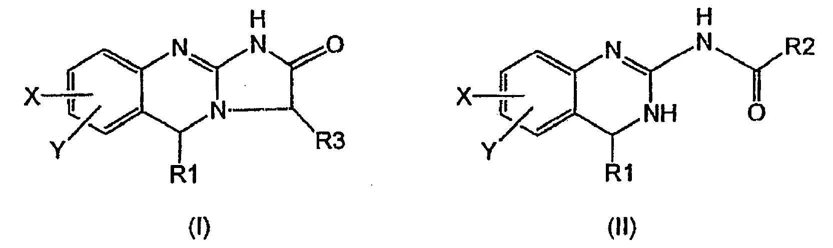 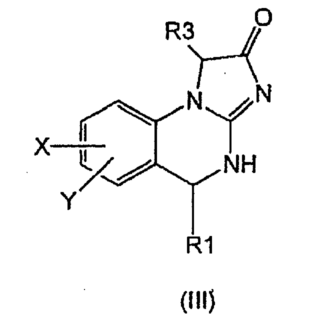 an equilibrating form thereof, a pharmaceutically acceptable salt thereof, or a pharmaceutically acceptable salt of an equilibrating form thereof,
wherein
R1 is H, C₁₋₆ alkyl, C₂₋₆ alkenyl, C₂₋₆ alkynyl, C₁₋₆ alkoxy, or C₆₋₁₀ aryl;
R2 is 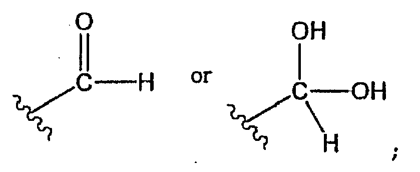 R3 is OH, Halogen, SH, O-C₁₋₆ alkyl or NH₂; and
X and Y are independently H or halogen.

Preferred embodiments include the X and Y substituents on the 5 and 6 positions of the benzyl ring.

In one embodiment, R1 is H or C₁₋₆ alkyl. In another embodiment, R1 is H or CH₃. In a preferred embodiment, R1 is H.

In one embodiment, R3 is OH or O-C₁₋₆ alkyl. In another embodiment, R3 is OH or OCH₃. In a preferred embodiment, R3 is OH.

In one embodiment, X is H, or halogen. In a preferred embodiment, X is H. In another preferred embodiment, X is Cl. In another embodiment, Y is H or Cl. In a preferred embodiment, Y is H. In another preferred embodiment, Y is C1.

Compounds in accordance with the present invention include 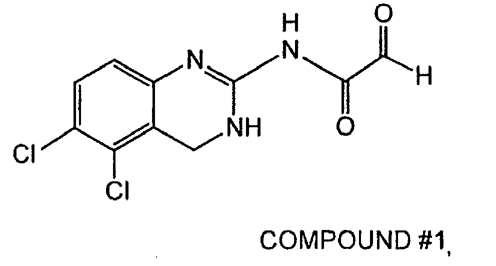 which is N-(5,6-dichloro-3,4-dihydroquinazolin-2-yl)-2-oxoacetamide; 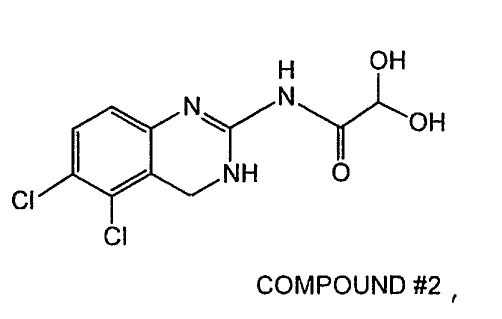 which is N-(5,6-dichloro-3,4-dihydroquinazolin-2-yl)-2-oxoacetamide hydrate; 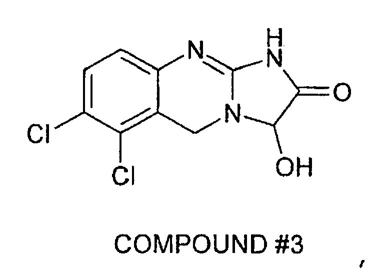 which is 6,7-dichloro-3-hydroxy-1,5-dihydro-imidazo[2,1-b]quinazolin-2-one; 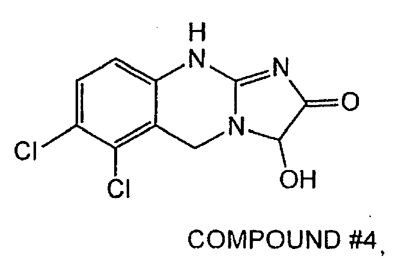 which is 6,7-dichloro-3-hydroxy-1,5-dihydro-imidazo[2,1-b]quinazolin-2-one;and 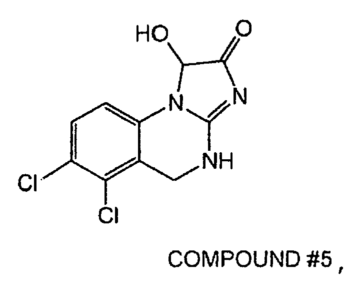 which is (6,7-Dichloro-1-hydroxy-3,5-dihydro-imidazo[1,2-a]quinazolin-2-one).

In accordance with the present invention, the compounds are administered to the patient in substantially pure form.

In accordance with the present invention, the compounds are in substantially pure form.

As used in this application, the term "substantially pure form" means that the compounds are at least 80% pure, preferably at least 85% pure, more preferably at least 90% pure, more preferably still at least 95% pure, and most preferably at least 99% pure as determined by standard analytical methods.

Without being bound to any theory (an understanding of the mechanism is not necessary to practice the present invention, and the present invention is not limited to any particular mechanism), the present inventors believe the compounds of the present invention are equilibrating forms. The equilibrating forms of the compounds of the present invention are depicted as follows:

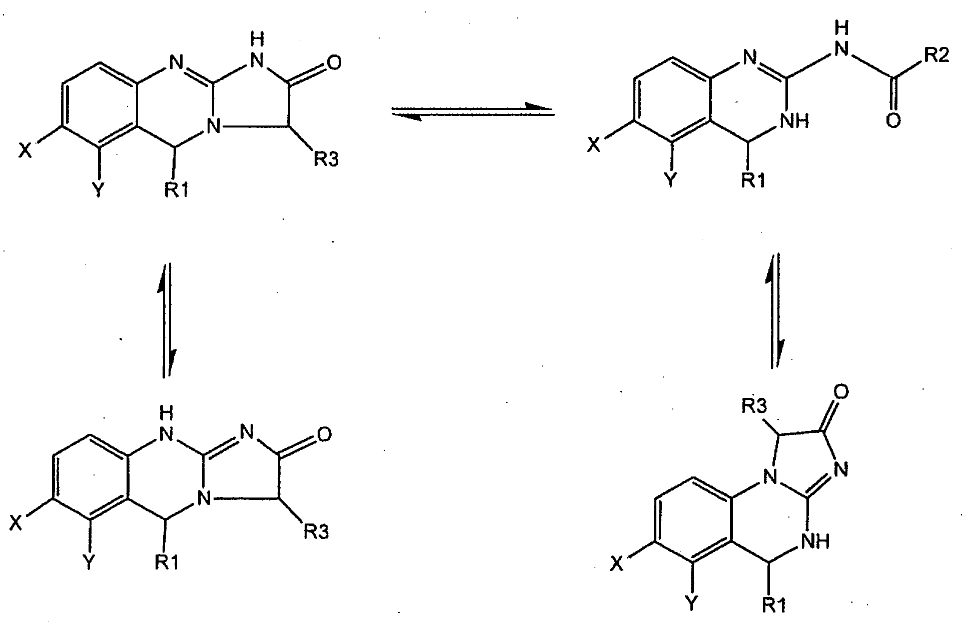

As used herein, the term "equilibrating form" includes tautomers of the compounds of formulas (I) through (III).

The equilibrating forms of the compounds of the invention can also be represented as follows:

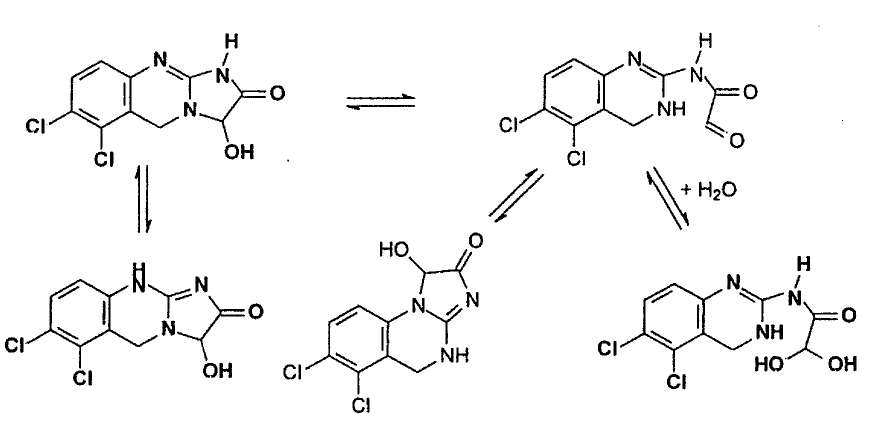

All such equilibrating forms are included in the scope of the present invention.

It will be appreciated by those of ordinary skill in the art that the compounds of formulas (I) through (III) may exist as tautomers or optical isomers. All equilibrating isomers and tautomers of such compounds are included in the scope of the present invention. The single optical isomer or enantiomer can be obtained by methods well known in the art, such as chiral HPLC, enzymatic resolution and chiral auxiliary, or can be stereoselectively synthesized.

There are also provided pharmaceutically acceptable salts of the compounds of the present invention. By the term "pharmaceutically acceptable salts" of the compounds of general formulas (I) through (III) those meant are those derived from pharmaceutically acceptable inorganic and organic acids and bases. Examples of suitable acids include hydrochloric, hydrobromic, sulphuric, nitric, perchloric, fumaric, maleic, phosphoric, glycollic, lactic, salicylic, succinic, toluene-p-sulphonic, tartaric, acetic, citric, methanesulphonic, formic, benzoic, malonic, naphthalene-2-sulphonic and benzenesulphonic acids. Other acids such as oxalic, while not in themselves pharmaceutically acceptable, may be useful as intermediates in obtaining the compounds of the invention and their pharmaceutically acceptable acid addition salts.

Salts derived from appropriate bases include alkali metal (e.g. sodium), alkaline earth metal (e.g. magnesium), ammonium, and NR₄+ (where R is C₁₋₄ alkyl) salts.

As used in this application, the term "alkyl" represents an unsubstituted or substituted (by a halogen, nitro, CONH₂, COOH, O-C₁₋₆ alkyl, O-C₂₋₆ alkenyl, O-C₂₋₆ alkynyl, hydroxyl, amino, or COOQ, wherein Q is C₁₋₆ alkyl, C₂₋₆ alkenyl; C₂₋₆ alkynyl) straight chain, branched chain or cyclic hydrocarbon moiety (e.g. isopropyl, ethyl, fluorohexyl or cyclopropyl). The term alkyl is also meant to include alkyls in which one or more hydrogen atoms is replaced by a halogen, more preferably, the halogen is fluoro (e.g. -CF₃ or - CH₂CF₃) .

As used herein, the terms "alkenyl" and "alkynyl" represent an alkyl (as defined above) containing at least one unsaturated group (e.g. allyl).

When there is a sulfur atom present, the sulfur atom can be at different oxidation levels: S, SO, or SO₂. All such oxidation levels are within the scope of the present invention.

Halogen herein means fluoro, chloro, bromo, and iodo.

The disclosure also provides methods of treating a subject (e.g., mammal, particularly humans) comprising administering to a subject in need of such treatment a therapeutically effective amount of at least one compound of formulas (I) through (III), formulation thereof, or unit dose forms thereof, each as described herein. The compounds of formulas (I) through (III) are used to inhibit cellular phosphodiesterase, particularly phosphodiesterases III and IV. More particularly, the compounds of formulas (I) through (III) are used to inhibit cellular phosphodiesterase III. The primary use for the compounds of formulas (I) through (III) is for the reduction of intermittent claudication in such subjects, typically manifested by an increased walking distance. The compounds of the present invention may also be used for the treatment of other disease states related to vasodilation including, for example, stroke and antiplatelet effects. Such active ingredients may also increase plasma high density lipoprotein cholesterol and apolipoprotein in subjects in need of such treatment as well as being used to treat sexual dysfunction.

"Treating" or "treatment" of a state, disorder or condition includes:
(1) preventing or delaying the appearance of clinical symptoms of the state, disorder or condition developing in a mammal that may be afflicted with or predisposed to the state, disorder or condition but does not yet experience or display clinical or subclinical symptoms of the state, disorder or condition,
(2) inhibiting the state, disorder or condition, i.e., arresting, reducing or delaying the development of the disease or a relapse thereof (in case of maintenance treatment) or at least one clinical or subclinical symptom thereof, or
(3) relieving the disease, i.e., causing regression of the state, disorder or condition or at least one of its clinical or subclinical symptoms.
In one embodiment, the present invention provides for treatment of intermittent claudication.

A "therapeutically effective amount" means the amount of a compound that, when administered to a subject for treating a state, disorder or condition, is sufficient to effect such treatment. The "therapeutically effective amount" will vary depending on the compound, the disease and its severity and the age, weight, physical condition and responsiveness of the subject to be treated.

A subject in need thereof is an individual, for example a human or other mammal that would benefit by the administration of the compounds of the present invention.

The benefit to a subject to be treated is either statistically significant or at least perceptible to the patient or to the physician.

It will be appreciated that the amount of a compound of the invention required for use in treatment will vary not only with the particular compound selected but also with the route of administration, the nature of the condition for which treatment is required, and the age and condition of the patient and will be ultimately at the discretion of the attendant physician or veterinarian. In general, however, a suitable dose will be in the range of from about 0.001 to about 50 mg/kg of body weight per day, preferably of from about 0.001 to about 5 mg/kg of body weight per day, more preferably of from about 0.001 to about desirably 0.5 mg/kg of body weight per day, or most desirably from about 0.001 to about 0.1 mg/kg of body weight per day. In further embodiments, the ranges can be of from about 0.1 to about 750 mg/kg of body weight per day, in the range of 0.5 to 60 mg/kg/day, and in the range of 1 to 20 mg/kg/day.

The desired dose may conveniently be presented in a single dose or as divided doses administered at appropriate intervals, for example as two, three, four or more doses per day. If the compounds are administered transdermally or in extended release form, the compounds could be dosed once a day or less.

The compound is conveniently administered in unit dosage form, for example containing 0.1 to 50 mg, conveniently 0.1 to 5 mg, or most conveniently 0.1 to 5 mg of active ingredient per unit dosage form. In yet a further embodiment, the compound can conveniently be administered in unit dosage form, for example containing 10 to 1500 mg, preferably 20 to 1000 mg, or more preferably 50 to 700 mg of active ingredient per unit dosage form.

Ideally the active ingredient should be administered to achieve peak plasma concentrations of the active compound of from about 0.01 to about 5µM, from about 0.01 to about 1µM, from about 1 to about 75µM, from about 2 to 50 µM, or from about 3 to about 30 µM. This may be achieved, for example, by the intravenous injection of a 0.1 to 5% solution of the active ingredient, optionally in saline, or orally administered as a bolus containing about 0.1 to about 5 mg or about 1 to about 500 mg of the active ingredient. Desirable blood levels may be maintained by a continuous infusion to provide about 0.0001 to about 1.0 mg/kg/hour or about 0.0001 to about 0.5 mg/kg/hour or by intermittent infusions containing about 0.001 to about 0.1 mg/kg of the active ingredient. In a further embodiment, desirable blood levels may be maintained by a continuous infusion to provide about 0.01 to about 5.0 mg/kg/hour or by intermittent infusions containing about 0.4 to about 15 mg/kg of the active ingredient. While it is possible that, for use in therapy, a compound of the invention may be administered as the raw chemical, it is preferable to present the active ingredient as a pharmaceutical formulation. The invention thus further provides a pharmaceutical formulation comprising a compound of formulas (I) through (III) or an analogue thereof together with one or more pharmaceutically acceptable carriers, and, optionally, other therapeutic and/or prophylactic ingredients. The carrier(s) must be "acceptable" in the sense of being compatible with the other ingredients of the formulation and not deleterious to the recipient thereof.

Pharmaceutical formulations include those suitable for oral, rectal, nasal, topical (including buccal and sublingual), transdermal, vaginal or parenteral (including intramuscular, sub-cutaneous and intravenous) administration or in a form suitable for administration by inhalation or insufflation. The formulations may, where appropriate, be conveniently presented in discrete dosage units and may be prepared by any of the methods well known in the art of pharmacy. All methods include the step of bringing into association the active compound with liquid carriers or finely divided solid carriers or both and then, if necessary, shaping the product into the desired formulation.

Pharmaceutical formulation suitable for oral administration may conveniently be presented as discrete units such as capsules, cachets or tablets (each containing a predetermined amount of the active ingredient); as a powder or granules; or as a solution, suspension or emulsion. The active ingredient may also be presented as a bolus, electuary or paste. Tablets and capsules for oral administration may contain conventional excipients such as binding agents, fillers, lubricants, disintegrants, or wetting agents. The tablets may be coated using methods well known in the art. Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, emulsifying agents, non-aqueous vehicles (which may include edible oils), or preservatives.

The compounds of the invention may also be formulated for parenteral administration (e.g. by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulatory agents such as suspending, stabilizing and/or dispersing agents. Alternatively, the active ingredient may be in powder form, obtained by aseptic isolation of sterile solid or by lyophilization from solution, for constitution with a suitable vehicle, e.g. sterile, pyrogen-free water, before use.
For topical administration to the epidermis, the compounds of the invention may be formulated as ointments, creams or lotions, or as a transdermal patch. Such transdermal patches may contain penetration enhancers such as linalool, carvacrol, thymol, citral, menthol or t-anethole. Ointments and creams may, for example, be formulated with an aqueous or oily base with the addition of suitable thickening, and/or gelling, agents. Lotions may be formulated with an aqueous or oily base and will in general also contain one or more emulsifying agents, stabilizing agents, dispersing agents, suspending agents, thickening agents, or coloring agents.

Formulations suitable for topical administration in the mouth include lozenges comprising active ingredient in a flavored base, usually sucrose and acacia or tragacanth; pastilles comprising the active ingredient in an inert base such as gelatin and glycerin or sucrose and acacia; and mouthwashes comprising the active ingredient in a suitable liquid carrier.

Pharmaceutical formulations suitable for rectal administration wherein the carrier is a solid are most preferably presented as unit dose suppositories. Suitable carriers include cocoa butter and other materials commonly used in the art, and the suppositories may be conveniently formed by admixture of the active compound with the softened or melted carrier(s) followed by chilling and shaping in moulds.

Formulations suitable for vaginal administration may be presented as pessaries, tampons, creams, gels, pastes, foams or sprays containing in addition to the active ingredient such carriers as are known in the art to be appropriate.

For intra-nasal administration the compounds of the invention may be used as a liquid spray or dispersible powder or in the form of drops. Drops may be formulated with an aqueous or non-aqueous base also comprising one more dispersing agents, solubilizing agents or suspending agents. Liquid sprays are conveniently delivered from pressurized packs.

For administration by inhalation the compounds of the invention are conveniently delivered from an insufflator, nebulizer or a pressurized pack or other convenient means of delivering an aerosol spray. Pressurized packs may comprise a suitable propellant such as dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount.

Alternatively, for administration by inhalation or insufflation, the compounds of the invention may take the form of a dry powder composition, for example a powder mix of the compound and a suitable powder base such as lactose or starch. The powder composition may be presented in unit dosage form in, for example, capsules or cartridges or, for example, gelatin or blister packs from which the powder may be administered with the aid of an inhalator or insufflator.

When desired, the above described formulations may be adapted to give sustained release of the active ingredient.

In a further embodiment, there is provided a combination comprising a compound in accordance with the present invention with one or more phosphodiesterase inhibitors. Various compounds are known as inhibitors of phosphodiesterases, including vinpocetine, milrinone, amrinone, pimobendan, cilostamide, enoximone, piroximone, vesnarinone, rolipram, R020-1724, zaprinast, dipyridamole, pentoxifylline, sildenafil citrate (ViagraΘ), doxazosin, papaverine, prazosin, terazosin, trimazosin and hydralazine. PCT Publication No. WO 94/28902 discloses a series of pyrazole [4,3-d]pyrimidin-7-ones cGMP phosphodiesterase inhibitors. PCT Publication No. WO 96/16644 also discloses a variety of cGMP phosphodiesterase inhibitors, including griseolic acid derivatives, 2-phenylpurinone derivatives, phenylpyridone derivatives, fused and condensed pyrimidines, a pyrimdopyrimidine derivative, a purine compound, a quinazoline compound, a phenylpyrimidone derivative, an imidazoquinoxalinone derivative or aza analogues thereof, a phenylpyridone derivative, and others.

The compounds of the present invention could also be used in combination with cilostazol (Pletal^{TM}).
The combinations referred to above may conveniently be presented for use in the form of a pharmaceutical formulation, and thus pharmaceutical formulations comprising a combination as defined above together with a pharmaceutically acceptable carrier therefore comprise a further embodiment of the invention.

The individual components of such combinations may be administered either sequentially or simultaneously in separate or combined pharmaceutical formulations.

When a compound of formulas (I) through (III) or an analogue thereof is used in combination with a second therapeutic agent, the dose of each compound may be either the same as or differ from that when the compound is used alone. Appropriate doses will be readily appreciated by those of ordinary skill in the art.

The ratio between the compounds of the present invention and the second therapeutic agent will be readily appreciated by those of ordinary skill in the art. For example, one may use from about 1:5000 to about 1:500, about 1:500 to about 1:100, about 1:1 to about 1:50, about 1:1 to about 1:30, about 1:1 to about 1:20, about 1:1 to about 1:15, about 1:1 to about 1:10, about 1:1 to about 1:5, or about 1:1 to about 1:3 of compounds of the invention:second therapeutic agent. If a further therapeutic agent is added, ratios will be adjusted accordingly.

The following examples are provided to illustrate various embodiments of the present invention and shall not be considered as limiting in scope.

### Example 1

### Preparation of Compound #1 of the present invention

The Compound #1 N-(5,6-dichloro-3,4-dihydroquinazolin-2-yl)-2-oxoacetamide (1) having an m/z of 271 was synthesized. Compound #1 may also exist as its hydrate (2).

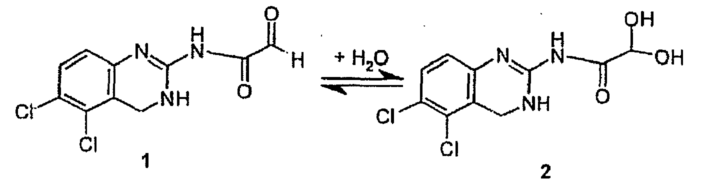

### Preparation of Compound #1

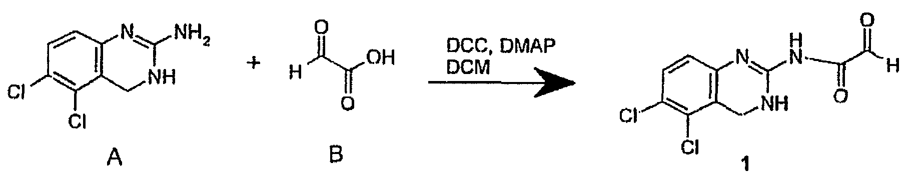

This approach to the required aldehyde (1) involved the reaction of 2-amino-5,6-dichloro-3,4-dihydroquinazoline (A) with glyoxalic acid hydrate (B) using a dicyclohexylcarbodiimide (DCC)-type coupling approach.

The coupling of (A) and glyoxalic acid (B) was conducted using dicyclohexylcarbodiimide (DCC), dimethylaminopyridine (DMAP) in dichloromethane (DCM). Product (A) can be obtained by methods known in the art (see, for example, US patent 6,194,420, issued February 27, 2001). A small amount of dimethylformamide (DMF) was added to aid the solubility of product (A). When these reagents were mixed in the dichloromethane solvent, a precipitate formed. This precipitate was filtered and the solvent evaporated. This crude reaction mixture was analyzed by LC-MS, showing 90% starting material and 7% of material with a mass of 271.

This reaction was performed on a 150 mg scale. The precipitate formed (170 mg) in this reaction was collected and analyzed along with the contents of the filtrate. By LC-MS analysis the precipitate was comprised of two main components, product (A) (25% by peak area) and the material with a mass of 271 (66% by peak area); however, NMR analysis of the same material indicated that the major component was, in fact, dicyclohexylurea (DCU), the reacted form of the coupling agent DCC. Attempted purification of this material by HPLC resulted in removal of the DCU (as determined by NMR analysis), but gave a mixture of (A) (18% by peak area) and the material of mass 271 (73% by peak area). However, only one aromatic peak containing product was observed in the NMR spectrum.

Successful purification of Compound #1 was achieved using reverse phase chromatography. Gradient elution starting from water to methanol gave three distinct fractions: Firstly (A), secondly the material of mass 271, and finally the DCU (as determined by NMR and HPLC analysis).

As shown above, Compound #1 is an equilibrating form of Compound #3 and Compound #4. Therefore, as stated above, under the conditions of these examples, it is believed that the compounds are interconverting and that Compound #3 and Compound #4 (both known as 3-hydroxyanagrelide) are also present.

### EXAMPLE 2

### Alternative Synthesis of Compound #1

### STEP 1

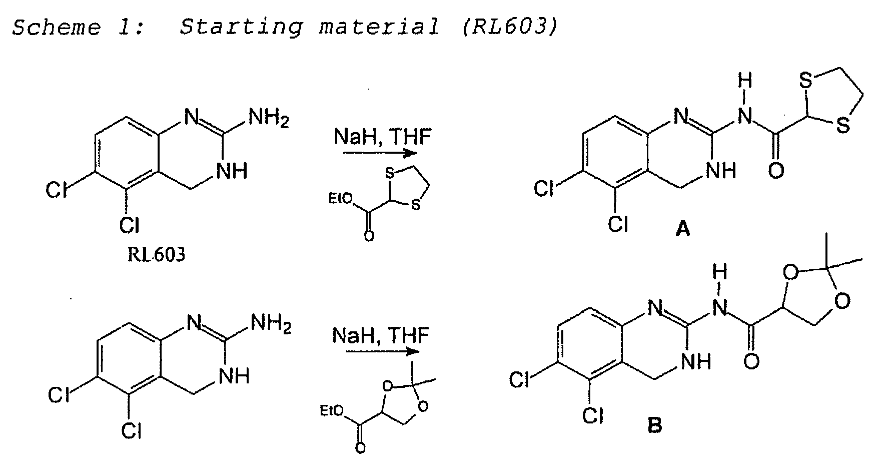

RL603 can be reliably acylated in modest to good yield by using 2.2 equivalents of sodium hydride and 1.1 equivalents of ethyl 2,3-isopropylidene glycerate to yield the product B (Scheme 1). The anion of RL603 was formed by heating with the sodium hydride in THF at 50° C for 30 min under an inert atmosphere. Then the mixture was cooled to room temperature, the ester was added, and the reaction stirred for 3 days. Quick purification and usage of the products is preferable in order to avoid formation of fluorescent oxidized impurities. Purification was achieved by normal aqueous extraction and chromatography on silica (eluting with 40 - 50% ethyl acetate / 60 - 50% petrol).

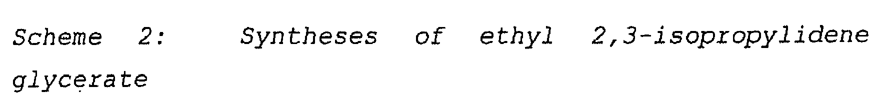

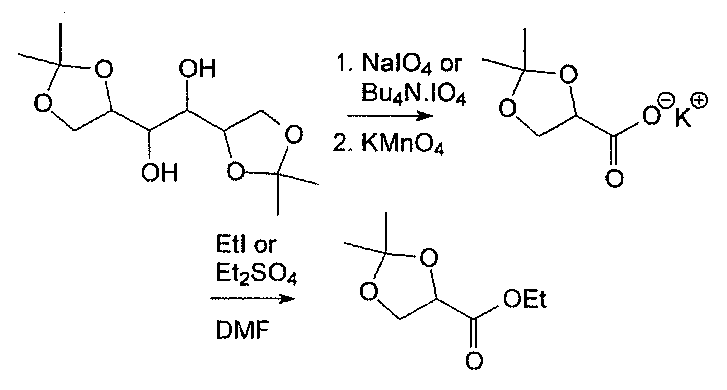

The ethyl 2,3-isopropylidene glycerate was prepared. 1,2:5,6-Di-isopropylidene-mannitol was treated with either sodium periodate or tetra-butylammonium periodate followed by potassium permanganate to yield crude potassium isopropylidene-glycerate, which can be purified by recrystallization from ethanol. This was treated with preferably iodoethane or diethyl sulfate (the use of the latter makes it more difficult to recover the product) in DMF to form the ethyl ester (Scheme 2). The ester was then distilled if necessary.

### STEP 2

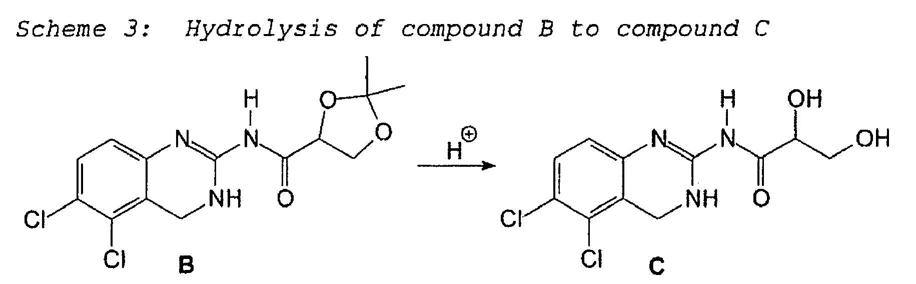

Treatment of the acetal-acylated RL603 derivative B with 0.1 M HCl in a 50:50 mixture of water:THF overnight resulted in a roughly equal mixture of RL603 and the desired diol (C) (scheme 3). The use of 5:3 water:trifluoroacetic acid for one hour very cleanly and selectively gives the desired diol (C), requiring no purification except removal of the solvents.

### STEP 3

### Conversion of compound (C) to Compound #1 and HPLC

Conversion of the diol (C) to Compound #1 was obtained by using sodium periodate in aqueous methanol or acetone. The diol was poorly soluble. Hydrolysis back to RL603 and formation of what appears to be an isomer of Compound #1 was noticed (it appears that this Iso-compound #1 is derived from the alternative mode of ring-closing of the intermediate aldehyde formed from periodate cleavage of diol (C).

Isomerisation of Compound #1 and *Iso*-compound #1 (a tautomer of Compound #5) are shown below:

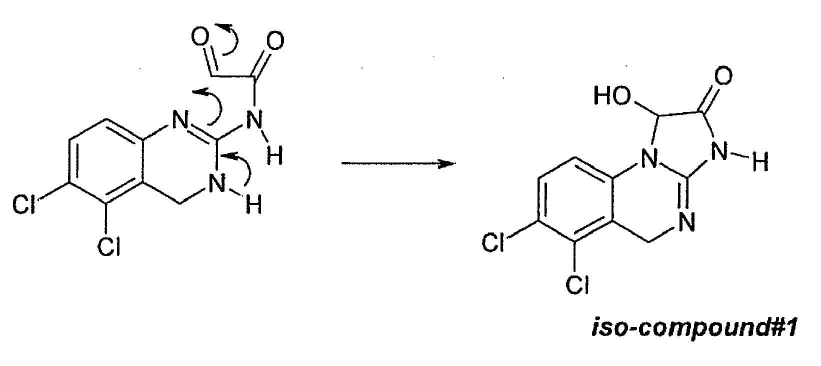

### Example 3

### Synthesis 6.7-dichloro-3-hydroxy-1,5-dihydro-imidazo[2,1-b]quinazolin-2-one (compound#3)

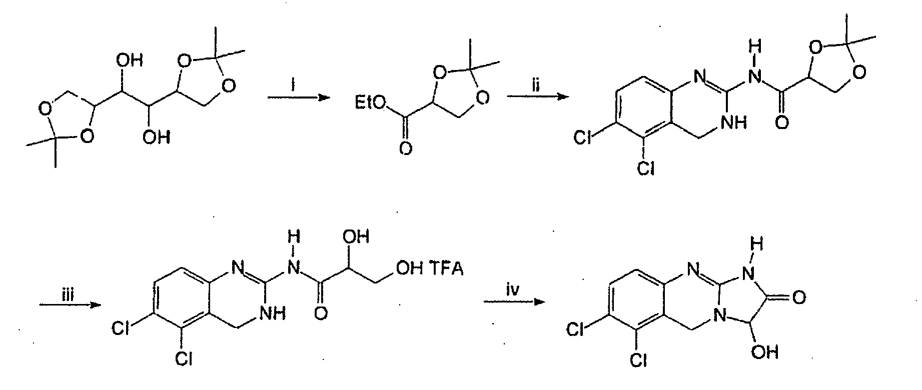

### Reagents and conditions:

Step (i): KMnO₄, KOH, water, room temperature (rt). 4 h, filter and evaporate, then DMF, ethyl iodide, rt, overnight aqueous work-up, 56% yield overall
Step (ii): 2-amino-5,6-dichloro-3,4-dihydroquinazoline, 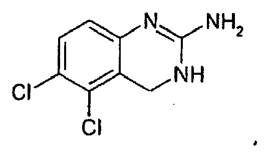 NaH, THF, 50 °C, 30 min, then rt, 48 h, aqueous work-up and column chromatography, 50% yield
Step (iii): CF₃CO₂H, water, rt, 1 h, evaporate, freeze-dry and triturate with ether, 100% yield
Step (iv): NaIO₄, pH 5 1 buffer, acetone, 10 °C, 20 min. evaporate, freeze-dry and column chromatography, 31% yield

### Purification

Chromatographic isolation of product (resolution from the isomer compound#5 (6,7-Dichloro-1-hydroxy-3,5-dihydro-imidazo[1,2-*a*]quinazolin-2-one was performed on normal phase silica in a glass column under compressed air pressure, eluting with a gradient of 0 - 10 % methanol / 100 - 90 % ethyl acetate The fractions were analyzed by TLC (thin-layer chromatography) eluting with THF (tetrahydrofuran) containing a few drops of concentrated ammonia
Analytical Data:
NMR
¹H NMR (300 MHz, DMSO-D₆): 7.47 (1H, d, *J* = 8.7 Hz), 6.96 (1H, d, *J* = 8.7 Hz). 6.91 (1H, d, *J=* 8 7 Hz), 5 01 (1H, d. *J*= 6.7 Hz), 4.58 + 4,47 (2H, *AB* system, *J=* 14 6 Hz)
¹³C NMR (75 MHz. DMSO-D₆): 130 00, 129 49, 125 32, 120 41, 113 05, 81.29, 41 89 (Weak sample, some signals not resolved )
*Infra Red Spectroscopy*
IR (neat): 1643, 1563, 1471 cm⁻¹
*Mass Spectroscopy*
(EI): 271 (M⁺, 100 %), 214 ( 86 %). 199 (34 %) %), 199 (35 %)

### Molecular weight determination

Hi-Res MS: Calc 270 991532 Found: 270 992371

### Melting point

M p: 170 °C (dec )

### Example 4

### Chemical synthesis of (6,7-Dichloro-1-hydroxy-3,5-dihydro-imidazo[1,2-a]quinazolin-2-one) Compound #5

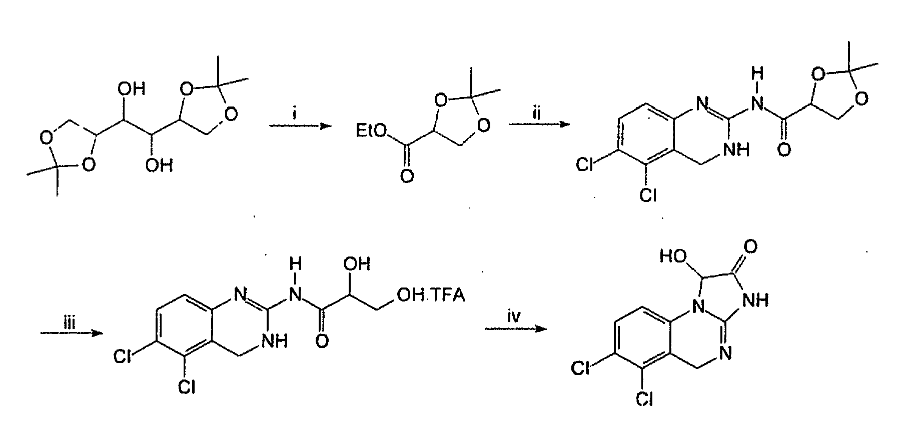

Reagents and conditions:
Step (i): KMnO₄, KOH, water. rt, 4 h, filter and evaporate. then DMF. ethyl iodide, rt, overnight. aqueous work-up, 56% yield overall.
Step (ii): RL603. NaH, THF, 50 °C, 30 min, then rt, 48 h. aqueous work-up and column chromatography, 50% yield
Step (iii): CF₃CO₂H, water, rt, 1 h, evaporate, freeze-dry and triturate with ether, 100% yield
Step (iv); NaIO₄, pH 10 8 buffer acetone, 10 °C, 20 min, evaporate, freeze-dry and column chromatography, 57% yield

Chromatography was performed on normal phase silica in a glass column under compressed air pressure, eluting with a gradient of 0 - 20 % methanol / 100 - 80% ethyl acetate in 2% increments Compound#3 (6,7-dichloro-3-hydroxy-1,5-dihydro-imidazo[2.1-b]quinazolin-2-one) eluted first followed by the Compound#5 The fractions were analysed by TLC (thin-layer chromatography) eluting with THF (tetrahydrofuran) containing a few drops of concentrated ammonia. Note that in this solvent system, Compound#5 elutes faster than the Compound#3, i e the reverse order of that from column chromatography
Analytical Data:
NMR
¹H NMR (300 MHz, DMSO-D₆): 9.40 (1H, s). 7.59 (1H, d, *J*= 8.8 Hz). 7 15 (1H. d. *J =* 9 2 Hz), 6.99 (1H, d, *J*= 8.8 Hz). 5.38 (1H, d, *J* = 9.2Hz). 4 62 + 4.56 (2H, *AB* system, *J =* 16 8 Hz).
¹³C NMR (75 MHz, DMSO-D₆): 182.90, 162.97, 134.02, 129.97, 129 26, 125.21. 118 89, 113.05. 79 87, 41 46
*Infra red spectroscopy*
IR (neat): 1653, 1472, cm⁻¹
*Mass spectrometry*
MS (EI): 271 (M⁺, 100 %), 214 (87 %), 199 (35 %)
*Molecular weight determination*
Hi-Res MS: Calc 270.991532 Found: 270 991783
Melting point
M p: 190 °C (dec)

### Example 5

### Evaluation of the effect of the compounds of this invention on phosphodiesterases

The profile of activity against a selection of phosphodiesterases (I-VI) was investigated. The methods used for the evaluation of the biological activity are all well known in the art. See for example, Weishaar et al; Biochem. Pharmacol., 35:787-800.

| **Effects of Compound#1 on various phosphodiesterases** | | | | |
|---|---|---|---|---|
| Assay Compound | Concentration | % inhibition | Reference compounds | |
| | | | IC₅₀ | (nH) |
| | | | | |
| 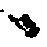 Phosphodiesterase I | | | 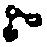 8-methoxy-IBMX | |
| Compound#1 | **10µM** | **26** | **2,410 nM** | *(1.1)* |
| | | | | |
| 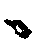 Phosphodiesterase II *(h)* | | | 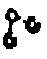 EHNA | |
| Compound#1 | **10uM** | **-** | **2,600 nM** | *(0.7)* |
| | | | | |
| 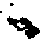 Phosphodiesterase III *(h)* | | | 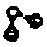 milrinone | |
| Compound#1 | **10uM** | **97** | **366 nM** | *(0.9)* |
| | | | | |
| 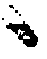 Phosphodiesterase IV *(h)* | | | 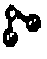 rolipram | |
| Compound#1 | **10uM** | **-** | **738 nM** | *(1.0)* |
| | | | | |
| 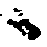 Phosphodiesterase V *(h)* | | | 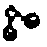 dipyridamole | |
| Compound#1 | **10uM** | **64** | **1,080 nM** | *(1.3)* |
| | | | | |
| 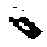 Phosphodiesterase VI | | | 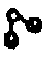 zaprinast | |
| Compound#1 | **10uM** | **89 g** | **839 nM** | *(0.8)* |
| | | | | |
| For the test compound(s), the results are expressed as a percent inhibition of control activity (mean values ; n = 2). | | | | |
| The symbol - indicates an inhibition of less than 10%. | | | | |

Compound #1 was screened, on two separate occasions, against human PDEIII and found to have an IC₅₀ between 0.69 and 1.1 nM (average 0.9 nM).

| **Effects of Compound#1 in the *in vitro* pharmacology assay and control value(s) for the reference compound(s)** | | | | | | |
|---|---|---|---|---|---|---|
| Assay Compound | IC₅₀ | KI | (nH) | Reference compound | | |
| | | | | IC₅₀ | Ki | (nH) |
| | | | | | | |
| 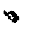 Phosphodiesterase III *(h)* | | | | 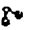 milrinone | | |
| Compound#1 (assay 1) | 0.69 nM | - | ***(1.1)*** | 267 nM | - | ***(0.9)*** |
| Compound#1 (assay 2) | 1.1 nM | - | ***(0.6)*** | 312 nM | - | ***(1.0)*** |

The IC₅₀ value against **PDEVI** was determined. This was found to be 360 nM (see below).

| **Effects of Compound #1 against PDEVI** | | | | | | | |
|---|---|---|---|---|---|---|---|
| Assay Compound | IC₅₀ | Ki | (nH) | Reference compound | | | |
| | | | | IC₅₀ | Ki | | (nH) |
| | | | | | | | |
| 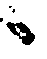 Phosphodiesterase VI | | | | 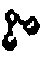 zaprinast | | | |
| Compound#1 | 360 nM | - | **(*1.0*)** | 1,120 nM | - | | ***(1,1**)* |

| **Effects of Compound #5 against PDEIII** | | | | | | | |
|---|---|---|---|---|---|---|---|
| Assay Compound | IC₅₀ | Ki | (nH) | Reference compound | | | |
| | | | | IC₅₀ | Ki | | (nH) |
| | | | | | | | |
| 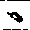 Phosphodiesterase III | | | | 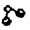 Milrinone | | | |
| Compound# | 1.8E-07 | - | *0.4* | 6.9E-07 | - | | *0.6* |

## Claims

1. A compound of formula 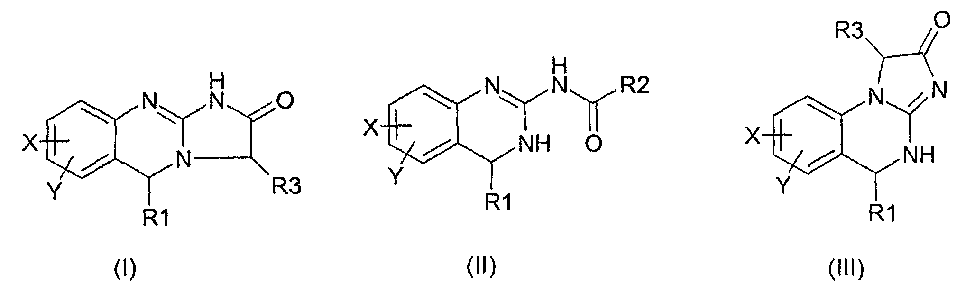 an equilibrating form thereof, a pharmaceutically acceptable salt of the compound or of the equilibrating form thereof,
wherein,
R1 is H, C₁₋₆ alkyl, C₂₋₆ alkenyl, C₂₋₈ alkynyl, C₁₋₆ alkoxy, or C₆₋₁₀ aryl;
R2 is 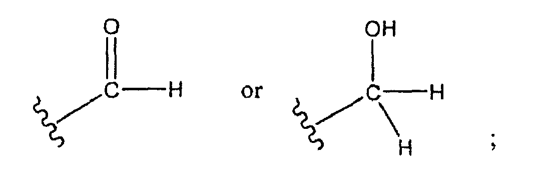 R3 is OH, Halogen, SH, O-C₁₋₆ alkyl or NH₂; and
X and Y are independently H or halogen, for use in increasing plasma high density lipoprotein and apolipoprotein levels or for use in the treatment of peripheral arterial disease, atherosclerosis, intermittent claudication, congestive heart failure, a disease related to increase platelet aggregation, thrombosis, stroke, or sexual dysfunction.

2. A compound for use according to claim 1, wherein the compound has the formula

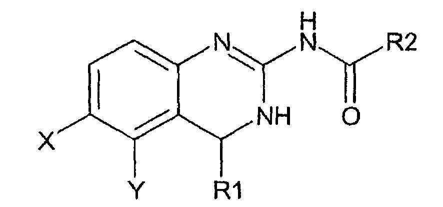

3. A compound for use according to claim 1, wherein R2 is

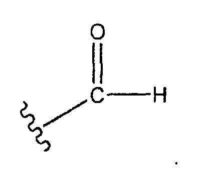

4. A compound for use according to claim 1, wherein R1 is H or C₁₋₆ alkyl,

5. A compound for use according to claim 1, wherein R1 is H.

6. A compound for use according to claim 1, wherein X is H or halogen.

7. A compound for use according to claim 1, wherein Y is H or Cl.

8. A compound for use according to claim 1, wherein X is Cl.

9. A compound for use according to claim 1, wherein Y is Cl.

10. A compound for use according to claim 1, wherein the compound is

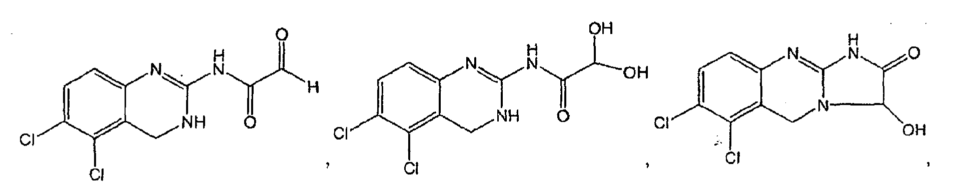

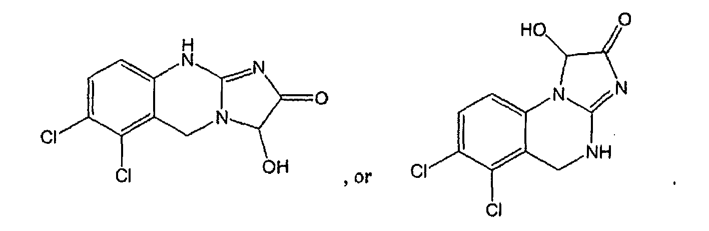

11. A compound of claim 1 for use in the treatment of peripheral arterial disease.

12. A compound of claim 1 for use in the treatment of atherosclerosis.

13. A compound of claim 1 for use in the treatment of intermittent claudication.

14. A compound of claim 1 for use in the treatment of congestive heart failure.

15. A compound of claim 1 for use in the treatment of stroke.

16. A compound of claim 1 for use in the treatment of sexual dysfunction.

## Patentansprüche

1. Verbindung der Formel 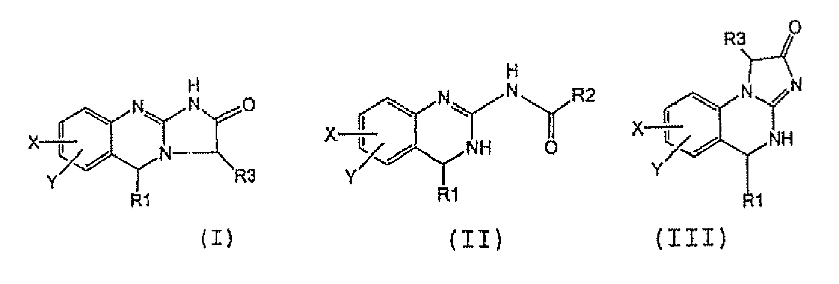 eine äquilibrierende Form davon, ein pharmazeutisch annehmbares Salz der Verbindung oder der äquilibrierenden Form davon,
in der
R1 H, C₁₋₆ Alkyl, C₂₋₆ Alkenyl, C₂₋₆ Alkinyl, C₁₋₆ Alkoxy oder C₆₋₁₀ Aryl ist;
R2 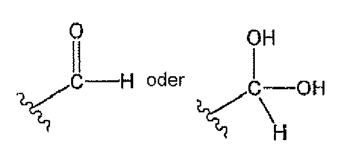 ist;
R3 OH, Halogen, SH, O-C₁₋₆ Alkyl oder NH₂ ist; und
X und Y unabhängig voneinander H oder Halogen sind, zur Verwendung beim Erhöhen der High Density Lipoprotein und Apolipoprotein Level im Plasma oder zur Verwendung in der Behandlung von Erkrankungen der peripheren Arterien, Atherosklerosis, Claudicatio Intermittens, dekompensierter Herzinsuffizienz, einer Erkrankung, die mit einer erhöhten Plättchenaggregation zusammenhängt, Thrombose, eines Schlaganfalls oder sexueller Dysfunktion.

2. Verbindung zur Verwendung nach Anspruch 1, wobei die Verbindung die folgende Formel aufweist

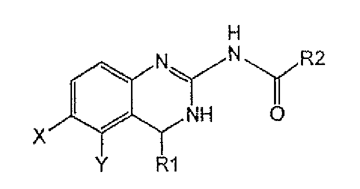

3. Verbindung zur Verwendung nach Anspruch 1, in der R2 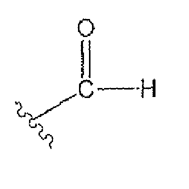 ist.

4. Verbindung zur Verwendung nach Anspruch 1, in der R1 H oder C₁₋₆ Alkyl ist.

5. Verbindung zur Verwendung nach Anspruch 1, in der R1 H ist.

6. Verbindung zur Verwendung nach Anspruch 1, in der X H oder Halogen ist.

7. Verbindung zur Verwendung nach Anspruch 1, in der Y H oder Cl ist.

8. Verbindung zur Verwendung nach Anspruch 1, in der X Cl ist.

9. Verbindung zur Verwendung nach Anspruch 1, in der Y Cl ist.

10. Verbindung zur Verwendung nach Anspruch 1, in der die Verbindung Folgendes ist

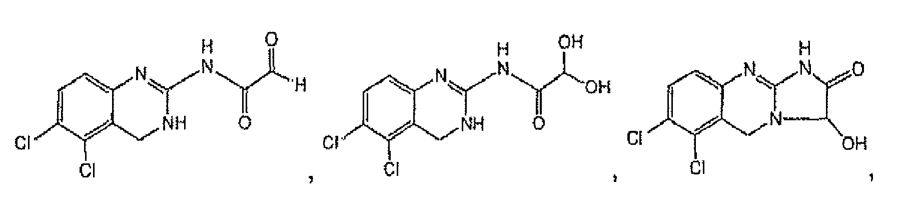

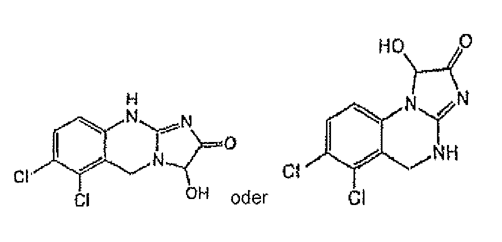

11. Verbindung nach Anspruch 1 zur Verwendung in der Behandlung einer Erkrankung der peripheren Arterien.

12. Verbindung nach Anspruch 1 zur Verwendung in der Behandlung von Atherosklerose.

13. Verbindung nach Anspruch 1 zur Verwendung in der Behandlung von Claudiocatio Intermittens.

14. Verbindung nach Anspruch 1 zur Verwendung in der Behandlung von dekompensierter Herzinsuffizienz.

15. Verbindung nach Anspruch 1 zur Verwendung in der Behandlung eines Schlaganfalls.

16. Verbindung nach Anspruch 1 zur Verwendung in der Behandlung von sexueller Dysfunktion.

## Revendications

1. Composé de formule : 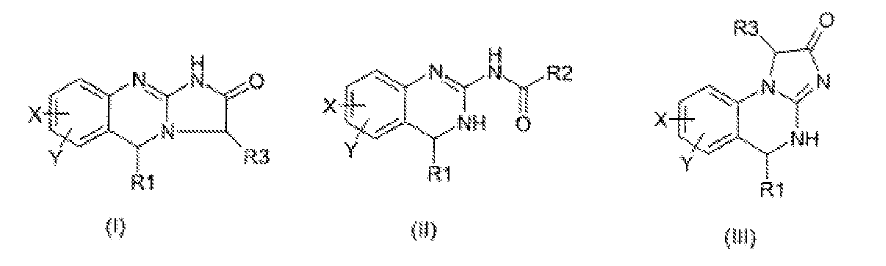 une forme équilibrante de celui-ci, un sel pharmaceutiquement acceptable du composé ou de la forme équilibrante de celui-ci,
formule dans laquelle :
- R1 représente H, alkyle en C₁₋₆, alcényle en C₂₋₆, alcynyle en C₂₋₆, alcoxy en C₁₋₆ ou aryle en C₆₋₁₀ ;
- R2 représente 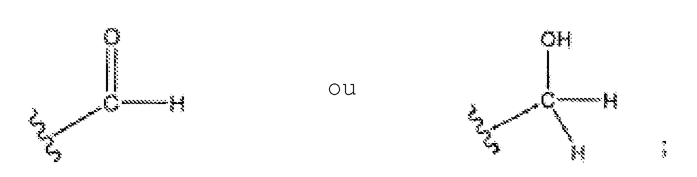
- R3 représente OH, halogène, SH, O-alkyle en C₁₋₆ ou NH₂ ; et
- X et Y représentent indépendamment H ou halogène,
pour une utilisation dans l'augmentation des taux de lipoprotéine et apolipoprotéine de densité élevée dans le plasma ou pour une utilisation dans le traitement de la maladie artérielle périphérique, de l'athérosclérose, de la claudication intermittente, de l'insuffisance cardiaque congestive, d'une maladie se rapportant à une agrégation plaquettaire accrue, la thrombose, l'accident vasculaire cérébral ou le dysfonctionnement sexuel.

2. Composé pour une utilisation selon la revendication 1, dans lequel le composé a la formule :

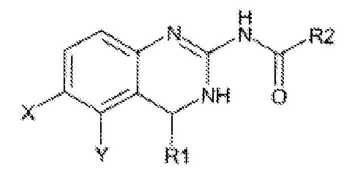

3. Composé pour utilisation selon la revendication 1, dans lequel R2 représente :

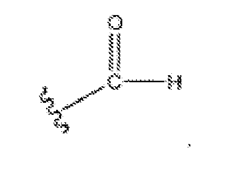

4. Composé pour utilisation selon la revendication 1, dans lequel R1 représente H ou alkyle en C₁₋₆.

5. Composé pour utilisation selon la revendication 1, dans lequel R1 représente H.

6. Composé pour utilisation selon la revendication 1, dans lequel X représente H ou halogène.

7. Composé pour utilisation selon la revendication 1, dans lequel Y représente H ou Cl.

8. Composé pour utilisation selon la revendication 1, dans lequel X représente Cl.

9. Composé pour utilisation selon la revendication 1, dans lequel Y représente Cl.

10. Composé pour utilisation selon la revendication 1, dans lequel le composé représente :

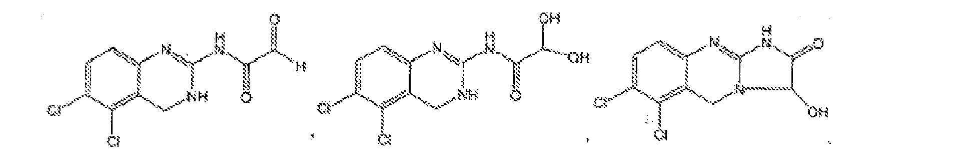

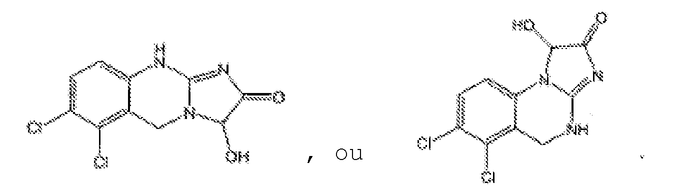

11. Composé selon la revendication 1 pour utilisation dans le traitement de la maladie artérielle périphérique.

12. Composé selon la revendication 1 pour utilisation dans le traitement de l'athérosclérose.

13. Composé selon la revendication 1 pour utilisation dans le traitement de la claudication intermittente.

14. Composé selon la revendication 1 pour utilisation dans le traitement de l'insuffisance cardiaque congestive.

15. Composé selon la revendication 1 pour utilisation dans le traitement de l'accident vasculaire cérébral.

16. Composé selon la revendication 1 pour utilisation dans le traitement du dysfonctionnement sexuel.
